# EUROPEAN PATENT SPECIFICATION

(11) **EP 1 415 648 B1**
(45) Date of publication and mention of the grant of the patent: **22.11.2006**
(21) Application number: 02746004.7
(22) Date of filing: 12.07.2002
(51) Int. Cl.: A61K 9/19, A61K 47/26, A61K 47/34, A61K 47/36

(54) **LYOPHILIZING COMPOSITION OF DRUG-ENCAPSULATING POLYMER MICELLE AND METHOD FOR PREPARATION THEREOF**
LYOPHILISIERENDE ZUSAMMENSETZUNG VON WIRKSTOFF-VERKAPSELNDER POLYMER-MIZELLE UND VERFAHREN ZU IHRER HERSTELLUNG
COMPOSITION DE LYOPHILISATION DE MICELLE POLYMERE D'ENCAPSULATION DE MEDICAMENTS ET PROCEDE DE PREPARATION ASSOCIE

(30) Priority: 13.07.2001 JP 2001213617; 13.07.2001 JP 2001213652
(43) Date of publication of application: 06.05.2004
(73) Proprietor: NanoCarrier Co., Ltd., Kashiwa-shi, Chiba-ken 277-0882 (JP)
(72) Inventor: OGAWA, Yasuaki, Otokuni-gun, Kyoto 618-0071 (JP); NAGASAKI, Shoko, Moriya-shi, Ibaraki 302-0128 (JP); NOGATA, Yoshihiko, Tagata-gun, Shizuoka 410-2123 (JP); SAGAWA, Katsuhiko, Shinmachi Namerikawa-shi,Toyama 936-0056 (JP); TSUCHIYA, Chieko, Funabashi-shi, Chiba 274-0072 (JP)
(74) Representative: Albrecht, Thomas
(86) International application number: PCT/JP2002/007099
(87) International publication number: WO 2003/005992

(56) References cited:
- WO-A-00/51564
- WO-A-01/05372
- WO-A-92/00081
- WO-A1-01/37879
- JP-A- 3 061 859
- JP-A- 11 335 267
- US-A- 6 143 276
- SON KYONGHEE ET AL.: 'Stabilization of teniposide in aqueous mixtures of detergent-phospholipid' PDA JOURNAL OF PHARMACEUTICAL SCIENCE AND TECHNOLOGY vol. 50, no. 6, 1996, pages 366 - 371, XP002957665

## Description

### Technical Field

The present invention relates to a preparation of a drug characterized by a specific physical form and a method for preparation thereof. The above physical form is a form of a core-shell type polymer micelle in which mainly a drug is encapsulated in a core part and in which a shell part comprises a hydrophilic polymer segment.

### Background Art

For the purpose of stably holding an active ingredient of medicine, the active ingredient is lyophilized and turned into a solid form. JP11/335267 describes a micelle made of a block copolymer which does not comprise additives to stabilize the polymer shell. However, the stability of the active ingredient is not yet the stability of the active ingredient is not yet satisfactory in a certain case in such operation or even in the resulting solid form. It is described in JP-11/125635-A that in order to stabilize a gold colloid-containing lyophilized product sensitizing protein (particularly an antibody), saccharides such as sucrose and β-cyclodextrin, threonine and aspartic acid are added to a sensitized gold colloid solution in lyophilization. Further, a lyophilized composition having the purpose of stabilizing an emulsion system regarded as containing a drug-encapsulating liposome using a phospholipid is described in JP-62/29513-A, and a solid carbohydrate which is pharmaceutically allowable is added to the above composition for the purposes of facilitating the reconstruction by water and enhancing the storage stability.

Thereafter, a drug-encapsulating liposome system using various modified phospholipids and a drug-encapsulating polymer micelle system using an amphiphilic block polymer have been proposed in order to achieve a specific drug delivery to the target. Both systems have intrinsic characteristics respectively, and therefore a large variety of the systems has been developed according to the purposes. It is known that in general, a polymer micelle system maintains an intermolecular micelle structure even when diluted to a so-called critical micelle concentration or lower and therefore has a solubilizing power as compared with the liposome system, so that it can stably be maintained to some extent.

As described above, it is said that a polymer micelle can relatively stably hold an encapsulated or sealed drug in a micelle, but from a practical point of view, the stability is not necessarily satisfactory in a state of an aqueous dispersion or solution of a micelle. Then, it is tried to lyophilize a polymer micelle solution. However, the polymer micelle particles are associated or coagulated in lyophilization due to various factors, and a growth in the particles and a deterioration in the resolubility in water are brought about in a certain case.

On the other hand, a large variety of methods is proposed as a method for preparing an aqueous dispersion or solution of such drug-encapsulating polymer micelle, but the aqueous dispersion or the solution obtained by any method has not been able to avoid causing the association or the coagulation described above between the polymer micelle particles when it is lyophilized as it is. The following typical methods for preparing a drug-encapsulating polymer micelle aqueous dispersion or solution (composition) are known.
a) Sealing method for a drug by stirring
   A water-scarcely soluble drug is dissolved, if necessary, in a water-miscible organic solvent, and the resulting solution is mixed with a block copolymer-dispersed aqueous solution by stirring. Heating in mixing by stirring makes it possible in a certain case to accelerate sealing of the drug in a polymer micelle.
b) Solvent volatilizing method
   A water-immiscible organic solvent solution of a water-scarcely soluble drug is mixed with a block copolymer-dispersed aqueous solution, and the organic solvent is volatilized while stirring.
c) Dialysis method
   A water-scarcely soluble drug and a block copolymer are dissolved in a water-miscible organic solvent, and then the resulting solution is dialyzed to a buffer solution and/or water using a dialysis membrane.
d) Others (not described in the official gazettes described above)

A water-scarcely soluble drug and a block copolymer are dissolved in a water-immiscible organic solvent, and the resulting solution is mixed with water and stirred to form an oil-in-water (O/W) type emulsion, followed by volatilizing the organic solvent.

Meanwhile, it is said that the respective methods described above have both merits and demerits. For example, in a) and b), an encapsulating rate of the drug into the polymer micelle is usually low; in c), the operation is complicated, and the polymer micelle can not be formed depending on the kind of the drug; and in d), the solution viscosity grows high depending on the kind of the block polymer and the kind of the drug, and the stirring operation is difficult in a certain case.

Accordingly, an object of the present invention is to provide a lyophilized preparation of a drug-encapsulating polymer micelle and which is inhibited particularly from association or coagulation between the polymer micelles and a composition which can conveniently be used for preparing such preparation.

### Disclosure of the Invention

The present inventors have found that even if a hydrophilic polymer segment is a drug-encapsulating polymer micelle system formed using a certain block copolymer comprising polyethylene glycol, the problems described above can be solved without exerting any adverse effect on the stability of the polymer micelle by carrying out lyophilization after adding polyethylene glycol and/or saccharides as a stabilizing agent.

Further, they have found that in producing a drug-encapsulating polymer micelle system (an aqueous dispersion or an aqueous solution), an aqueous dispersion or an aqueous solution of a drug-encapsulating polymer micelle can efficiently be obtained by preparing an aqueous solution of a block copolymer containing polyethylene glycol and/or saccharides and, if necessary, inorganic salts and a solution of a drug dissolved in a water-insoluble organic solvent and mixing and stirring both solutions thus obtained and that a lyophilized product showing an excellent solubilizing property without bringing about the problems described above, that is, association or coagulation between the polymer micelle particles is obtained by lyophilizing such dispersion or aqueous solution as it is.

Hence, according to the present invention, provided is an aqueous composition comprising a drug-encapsulating polymer micelle for preparing a lyophilized preparation of the drug-encapsulating polymer micelle, wherein:
(A) the composition further comprises at least one stabilizing agent selected from the group consisting of saccharides and polyethylene glycol and
(B) the above drug-encapsulating polymer micelle originates in a block copolymer having in a molecule, a hydrophilic polymer segment and a polymer segment which is hydrophobic or chargeable or which comprises the repetitive units of both of them, and it is a substantially spherical core-shell type micelle in which the drug is encapsulated principally in a core part and in which a shell part is constituted by the above hydrophilic polymer segment.

Provided as the present invention of a different embodiment is a drug-encapsulating polymer micelle preparation staying in a lyophilized form, wherein:
(a) the preparation comprises at least one stabilizing agent selected from the group consisting of saccharides and polyethylene glycol as an additional component,
(b) the above drug-encapsulating polymer micelle is formed from a block copolymer having in the molecule, a hydrophilic polymer segment and a hydrophobic or chargeable polymer segment or a polymer segment comprising the repetitive units of both of them, and it is a core-shell type micelle in which the drug is carried principally in a core part and in which a shell part is constituted by the above hydrophilic polymer segment and
(c) a drug-encapsulating polymer micelle solution which is homogeneously dispersed or solubilized is formed when the preparation is mixed with an aqueous medium.

Provided as the present invention of a further different embodiment are a novel process for producing a drug-encapsulating polymer micelle which can conveniently be utilized for preparing the aqueous composition and the drug-encapsulating polymer micelle preparation staying in a lyophilized form each described above, comprising the steps of:
(A) preparing an aqueous dispersion comprising a block copolymer having a hydrophilic segment and a hydrophobic or chargeable polymer segment or a polymer segment comprising the repetitive units of both of them and at least one additive selected from the group consisting of saccharides, inorganic salts and polyethylene glycol,
(B) preparing an organic solution of a fat-soluble drug using a water-immiscible organic solvent and
(C) mixing the aqueous dispersion and the organic solution each obtained in the step (A) and the step (B) and volatilizing the organic solvent while stirring the mixed solution thus obtained to prepare an aqueous dispersion or an aqueous composition of a drug-encapsulating polymer micelle, and a production process for a drug-encapsulating polymer micelle preparation staying in a lyophilized form, comprising as an additional step, a step of lyophilizing the aqueous dispersion or the aqueous solution of the drug-encapsulating polymer micelle obtained in the step (C) described above.

### Best Mode for Carrying Out the Invention

The "drug-encapsulating polymer micelle" referred in the present invention is a molecular aggregate in which a block copolymer is associated in an aqueous medium and is a structural matter (or a particulate matter) staying in a state in which the drug is sealed or carried in an intramolecular micelle structure (mainly a core part). Usually, it is substantially spherical. When referred to as "substantially spherical" in the present specification, it means that at least 80 %, preferably 90 % or more and more preferably 98 % or more of a particulate matter is spherical. Such drug-encapsulating polymer micelle maintains an intramolecular micelle structure even after diluted and can be present in an aqueous medium in a solubilizing state. The "aqueous medium" described above means water including deionized water, distilled water and sterilized water, buffer or isotonic water or a mixed solvent of a water-miscible organic solvent (for example, ethanol, acetone, acetonitrile, tetrahydrofuran and dimethylforamide) and water. The "aqueous composition" means a composition in which a drug-encapsulating polymer micelle stays in a solubilizing or dispersing state using the "aqueous medium" described above as a solvent or a dispersant. The aqueous composition stays preferably in a state containing substantially no organic solvent.

A block copolymer comprising a hydrophilic polymer segment (hereinafter referred to as the segment A) and a hydrophobic or chargeable polymer segment or a polymer segment comprising the repetitive units of both of them (hereinafter referred to as the segment B) can be used as a block copolymer which can form such polymer micelle. Such block copolymer includes "segment A-segment B" (AB type) and "segment A-segment B-(segment A)ᵢ" (wherein i is an integer of 1 or more). However, the AB type can be given as the preferred block copolymer.

A polymer constituting the segment A shall not be restricted, and polyethylene glycol (or polyoxyethylene), polysaccharide, polyvinylpyrrolidone and polyvinyl alcohol can be given. Among them, a polyethylene glycol segment can be given as the preferred segment. In general, the segment comprising 10 to 2500 repetitive units of oxyethylene is preferred, though shall not be restricted. The segment A may have any low molecular functional group or a molecular part (for example, a lower alkyl group, an amino group, a carboxyl group and a saccharide group, and among them, preferably a protein residue) at an end side opposite to a bonding end with the segment B as long as an adverse effect is not exerted in forming the polymer micelle.

On the other hand, the hydrophobic segment of the segment B shall not be restricted, and capable of being given are polyamino acid ester (polyaspartic acid ester, polyglutamic acid ester or partially hydrolyzed products thereof), poly(meth)acrylic acid ester, polylactide and polyester. Also, polyamines (for example, poly-di-lower alkylaminoalkylene (meth)acrylate), polyaspartic acid and polyglutamic acid can be given as the chargeable segment.

The AB type or ABA type block copolymer comprising such segment can form a polymer micelle by itself (no drug) in an aqueous medium if the segment B contained therein is a hydrophobic segment. If a polymer micelle is formed in the coexistence of a fat-soluble drug, the drug is encapsulated or sealed in the polymer micelle, particularly a core part formed by a hydrophobic segment. On the other hand, if the segment B is a chargeable segment (for example, polyamine), a polymer micelle can usually be formed by an interaction with a drug (for example, oligo- or polynucleotide, to be specific, ribozime, oligo DNA such as antisense DNA, RNA or peptide) which can be charged to a charge reverse to that of polyamine. The segment B can have the low molecular functional group or the molecular part each described above as long as an adverse effect is not exerted on the interaction of the drug with the segment B when a polymer micelle is formed at an end opposite to a bonding end with the segment A.

Polymers themselves or polymers derived from them described in, for example, JP-2777530-B (or US-5,449,513-B), WO96/32434, WO96/33233, WO97/06202 and Kataoka K. et al., Macromolecules, 1999, 32, 6892 to 6894 can be given as the typical ones of the block copolymer described above.

The typical example of the bloc copolymer in which the segment A contains a polyethylene glycol segment and in which the segment B comprises a polyamino acid ester (in a certain case, -CO-polyamino acid) segment can be represented, though not restricted, by the following Formula (I) or (II): 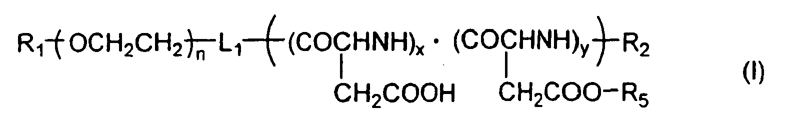 or 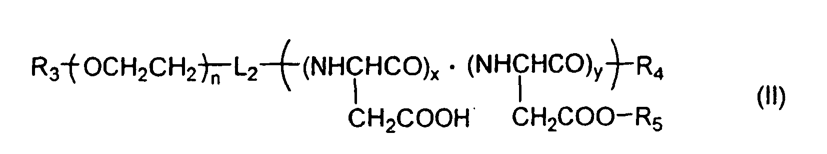 wherein
R₁ and R₃ each represent independently a hydrogen atom or a lower alkyl group substituted or not substituted with a functional group which may be protected;
R₂ represents a hydrogen atom, a saturated or unsaturated C₁ to C₂₉ aliphatic carbonyl group or an arylcarbonyl group;
R₄ represents a hydroxyl group, a saturated or unsaturated C₁ to C₃₀ aliphatic oxy group or an aryl-lower alkyloxy group;
R₅ represents a phenyl group, a C₁ to C₄ alkyl group or a benzyl group;
L₁ and L₂ each represent independently a linkage group;
n is an integer of 10 to 2500;
x and y are different or the same and are an integer in which the total of them is 10 to 300; either one of x and y is 0 or x to y falls in a range of 7 : 3 to 1 : 3; and when both are present, x and y each are present at random. The functional group allowed to be protected includes a hydroxyl group, an acetal group, a ketal group, an aldehyde group, a sucrose residue. When R₁ and R₃ represent a lower alkyl group which is substituted with a functional group allowed to be protected, the hydrophilic segment can be formed according to the methods described in WO96/33233, WO96/32434 and WO97/06202.
The linkage group can be changed principally according to the production process of the block copolymer and therefore shall not be restricted. To be specific, L₁ is a group selected from the group consisting of -NH-, -O-, -O-Z-NH-, -CO-, -CH₂-, -O-Z-S-Z- and -OCO-Z-NH- (wherein Z is independently a C₁ to C₄ alkylene group), and L₂ is a group selected from the group consisting of -OCO-Z-CO-and -NHCO-Z-CO- (wherein Z is a C₁ to C₄ alkylene group).

The aqueous composition for preparing a lyophilized preparation of a drug-encapsulating polymer micelle according to the present invention can be obtained by adding a stabilizing agent in preparing a polymer micelle under the coexistence of the block copolymer and the drug each described above according to a conventionally known method (for example, the methods described in the publications described above) or after preparing the polymer micelle and, if necessary, after exchanging an aqueous medium for solubilizing or dispersing the polymer micelle and, if necessary, by homogeneously mixing them. Accordingly, the above composition usually contains the drug-encapsulating polymer micelle and the stabilizing agent in the aqueous medium.

The stabilizing agent which can be used in the present invention may be a combination of at least one selected from the group consisting of any saccharides and polyethylene glycol. Such saccharides shall not be restricted, and maltose, trehalose, xylitol, glucose, sucrose, fructose, lactose, mannitol and dextrin can be given. On the other hand, polyethylene glycol having 4 to 5000, preferably 10 to 2500, more preferably 20 to 800 and particularly preferably 20 to 200 oxyethylene (that is, -(OCH₂CH₂)-) units can be given as polyethylene glycol. Macrogol 1000, 1540, 4000, 6000, 20000 and 35000 each described in, for example, a medical additive cyclopedia can be used for such polyethylene glycol.

In the present specification, the term of "poly" is used when referring to polyethylene glycol, the segment A and the segment B, and it is understood that the meaning of so-called "oligo" is included as well therein in a suited example as can be seen in the example of polyethylene glycol described above.

In the foregoing composition of the present invention, polyethylene glycol alone (allowed to contain a plurality of polyethylene glycols described above having different molecular weights) or a combination of polyethylene glycol and saccharides in a proportion of 1 to 0.1 : 0.1 to 1 in terms of a weight ratio is added as the stabilizing agent. In respect to an addition proportion of the drug-encapsulating polymer micelle to the stabilizing agent, the suitable proportion thereof is varied depending on the kinds of the drug-encapsulating polymer micelle and the stabilizing agent and therefore can not be restricted, and a proportion of the micelle thereto is usually 1 to 0.1 : 0.01 to 1 in terms of a weight of the block copolymer used.

When a concentration (in terms of a polymer weight) of the drug-encapsulating polymer micelle in the above composition is 1 to 90 (weight) %, a concentration of polyethylene glycol added to the micelle solution which is such composition is preferably 0.5 to 10 % by weight. On the other hand, a concentration of saccharides is 0 to 15 % by weight (when added, it can be 0.5 to 15 % by weight). Further, such composition is preferably adjusted to a pH of 4.0 to 7.5 from the viewpoint of subsequent lyophilization. Accordingly, the above composition can contain a buffering agent, salts and an antioxidant (for example, ascorbic acid, ascorbates and thiosulfates).

The drug which is encapsulated or sealed in the drug-encapsulating polymer micelle described above may be any drug as long as they are such drugs as can achieve the objects of the present invention, and drugs falling in a category of a fat-soluble drug can usually be given. In this case, the term "fat-soluble" means a property of a compound which can be dissolved in, for example, an organic solvent such as dichloromethane, diethyl ether and ethyl acetate capable of being applied to a production process for a drug-encapsulating polymer micelle described later, and it means as well a property of a compound which can be dissolved in a mixed solvent of dimethylformamide and dimethylsulfoxide.

The examples of the fat-soluble drug include, though not restricted, anticancer drugs comprising paclitaxel, topotecan, camptothecine, cisplatin, daunorubicin, methotrexate, mitomycin C, docetaxel, binclestin and derivatives thereof, polyene base antibiotics, for example, anphoterisin B and nystatin and in addition thereto, fat-soluble drugs such as prostaglandins and derivatives thereof. Among them, paclitaxel, topotecan and docetaxel are strongly intended to be used in the present invention.

The drug-encapsulating polymer micelle described above may be obtained by a conventionally known production process as described above, and it can conveniently be obtained as well by the following production process for a drug-encapsulating polymer micelle which is another embodiment of the present invention.

According to the production process of the above present invention, prepared is an aqueous dispersion comprising the block copolymer described above and at least one additive selected from the group consisting of saccharides, inorganic salts and polyethylene glycol. Saccharides and polyethylene glycol which can be used as the additive can be the same as those given as the examples of the "stabilizing agent" described above. On the other hand, any compounds can be used as the inorganic salts in the present invention as long as they meet the objects of the present invention and are pharmaceutically allowable, and the preferred salts include chlorides such as sodium chloride, potassium chloride, magnesium chloride and calcium chloride.

The aqueous dispersion described above can be prepared by adding the block copolymer and the respective additives to water at the same time and stirring them or preparing in advance the aqueous solution of the additives and adding the block copolymer thereto, or preparing a mixture in an inverse order to the above and stirring and mixing it. A supersonic wave as well as conventional stirrers may be used for stirring. Such dispersion shall not be restricted, and capable of being usually added are the block copolymer in a concentration of 0.1 to 40 % by weight, the saccharides in a concentration of 0.5 to 15 % by weight, polyethylene glycol in a concentration of 0.5 to 10 % by weight and the inorganic salts in a concentration of 0.5 to 10 % by weight.

According to the present invention, an organic solution in which the drug described above is dissolved in a water-immiscible organic solvent is prepared. Such solvent shall not be restricted and includes dichloromethane, chloroform, diethyl ether, dibutyl ether, ethyl acetate, butyl acetate and mixed solvents thereof. A suitable drug concentration in the above solution is varied depending on the combination of the solvent and the drug used, and it can usually be a concentration of 0.1 to 10 % by weight. The mixing operation described above can be carried out at a room temperature or a lower temperature.

Both of the aqueous dispersion and the organic solution thus prepared are mixed at one time or the latter is slowly added to the former, or a reverse procedure thereto is carried out to prepare a mixed solution, and the mixed solution is subjected to stirring treatment (including supersonic treatment) for enough time for the drug to be encapsulated or sealed in a polymer micelle. Such treatment is better carried out at a room temperature or a lower temperature (about 5°C). The organic solvent may be volatilized through the stirring treatment.

A drug-encapsulating polymer micelle dispersion is obtained by the operations described above, and saccharides and polyethylene glycol are added, if necessary, to the above dispersion as described above, whereby the drug-encapsulating polymer micelle can be stabilized in, for example, lyophilization treatment which shall be carried out subsequently or coagulation between the micelle particles can be inhibited. Saccharides and/or polyethylene glycol are preferably added so that the respective final concentrations thereof based on the total weight of the drug-encapsulating polymer micelle composition are 0.1 to 15 % by weight in the case of saccharides and 0.5 to 10 % by weight in the case of polyethylene glycol, considering whether or not they are added in preparing the drug-encapsulating polymer micelle dispersion described above. However, they may be added in such concentrations as exceeding the concentrations described above as long as an adverse effect is not exerted in preparing the lyophilized product of the drug-encapsulating polymer micelle and restructuring the resulting lyophilized product in an aqueous medium. Further, a pH in preparing the preparation of the present invention is preferably 4.0 to 7.5, and a pH controlling agent and an antioxidant (ascorbic acid, sodium ascorbate and sodium thiosulfate) can be added if necessary.

In the production process of the present invention described above in details, the raw materials and the additives used are common to those of the aqueous composition of the present invention as described above. Accordingly, the drug-encapsulating polymer micelle dispersion obtained by the above production process can be the above aqueous composition as it is.

The drug-encapsulating polymer micelle dispersion or the aqueous composition of the present invention produced according to the production process of the present invention can provide a lyophilized drug-encapsulating polymer micelle preparation by a normal process for lyophilization, for example, by freezing the above liquid composition at -1 to -60°C and then drying it under reduced pressure. The drug-encapsulating polymer micelle preparation thus obtained having a lyophilized form falls as well in one embodiment of the present invention. Such drug-encapsulating polymer micelle preparation forms a homogeneously dispersed or solubilized drug-encapsulating polymer micelle solution when mixed with an aqueous medium. Further, an average particle diameter of the above micelle present in the above solution (restructure after lyophilization) is scarcely different from an average particle diameter of the drug-encapsulating polymer micelle present in the composition described above before lyophilization, or if different, it usually grows large up to about twice, and nothing more.

The present invention shall be explained below in further details with reference to specific examples, but the present invention shall not be intended to be restricted to these examples.

### Example 1 (investigation of effect exerted by adding saccharides in void micelle)

Polyethylene glycol (molecular weight: 12000)-co-50 % partially hydrolyzed polybenzyl aspartate (n = 50) (hereinafter referred to as PEG-PBLA12-50. PH. 50 %) 500 mg was weighed in a screw tube bottle, and 50 mL of dichloromethane was added thereto and stirred to dissolve it. Next, the dichloromethane solution was concentrated up to 5 mL by blowing nitrogen gas, and 50 mL of water was added thereto and vigorously stirred for 30 minutes. Then, the stopper was opened, and the solution was stirred in a cold place for a whole day and night to prepare a polymer micelle. Then, supersonic treatment was carried out, and various saccharides shown in Table 1 were added and dissolved in a concentration of 40 to 160 mg/mL.
The solution was frozen in a dry ice-acetone freezing mixture to prepare a lyophilized preparation. Further, a preparation in which no saccharides were added was prepared as a comparative lyophilized preparation.

A micelle solution before lyophilization and a micelle solution obtained by lyophilizing the micelle solution and then redissolving it in water were measured for a particle size by means of a dynamic light scattering particle size meter (DLS-7000DH type, manufactured by Ohtsuka Electron Co., Ltd.), and the resolubility after lyophilization was visually evaluated after adding 10 mL of water to 50 mg of the lyophilized product. (Evaluation criteria; good: redissolved in shorter than 15 seconds when lightly shaken by a hand at a room temperature, average: redissolved in 15 seconds or longer and shorter than 2 minutes when lightly shaken by a hand at a room temperature, bad: redissolved in 2 minutes or longer or partially not redissolved when lightly shaken by a hand at a room temperature, and a block remained). The results thereof are shown in Table 1.

PEG-PBLA12-50. PH. 50 % can be shown by the following formula:

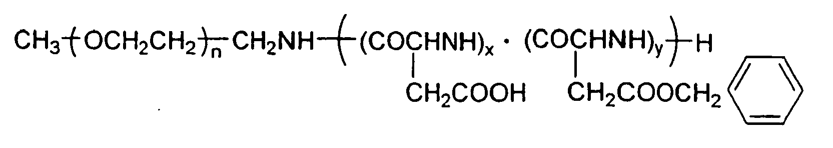

**Table 1 Average particle diameter change ratio before and after lyophilization in adding saccharides in a void micelle and resolubility**

| Additives | Additive concentration | Particle diameter before lyophilization | Particle diameter after lyophilization | Average particle diameter change ratio before & after lyophilization | Resolubility |
|---|---|---|---|---|---|
| | (mg/mL) | (nm) | (nm) | | |
| Maltose | 40 | 94.3 | 118.5 | 1.26 | Average |
| Maltose | 50 | 91.8 | 136.0 | 1.48 | Average |
| Maltose | 100 | 99.3 | 264.3 | 2.66 | Average |
| Trehalose | 40 | 104.6 | 128.0 | 1.22 | Average |
| Trehalose | 80 | 85.4 | 133.8 | 1.40 | Average |
| Trehalose | 160 | 104.4 | 287.1 | 2.75 | Average |
| Xylitol | 40 | 90.1 | 113.6 | 1.24 | Average |
| Glucose | 40 | 99.1 | 150.5 | 1.52 | Average |
| Glucose | 80 | 104.3 | 279.5 | 2.68 | Average |
| Glucose | 160 | 94.1 | 253.6 | 2.70 | Average |
| Sucrose | 40 | 93.1 | 145.6 | 1.56 | Average |
| Sucrose | 80 | 107.6 | 143.3 | 1.33 | Average |
| Mannitol | 40 | 98.5 | 146.8 | 1.49 | Average |
| Dextrin | 40 | 128.6 | 300.3 | 2.34 | Average |
| Not added | - | 95.6 | 3269 | 34.2 | Bad |

### Example 2 (investigation of effect exerted by adding Macrogols in void micelle)

PEG-PBLA12-50. PH. 50% 500 mg was weighed in a screw tube bottle, and 50 mL of dichloromethane was added thereto and stirred to dissolve it. Next, the dichloromethane solution was concentrated up to 5 mL by blowing nitrogen gas, and 50 mL of water was added thereto and vigorously stirred for 30 minutes. Then, the stopper was opened, and the solution was vigorously stirred in a cold place for a whole day and night to prepare a polymer micelle. Thereafter, supersonic treatment was carried out, and various Macrogols shown in Table 2 were added and dissolved in a concentration of 20 mg/mL. The solution was frozen in a dry ice-acetone freezing mixture to prepare a lyophilized preparation.

A micelle solution before lyophilization and a micelle solution obtained by lyophilizing the micelle solution and then redissolving it in water were measured for a particle size by means of the dynamic light scattering particle size meter (DLS-7000DH type, manufactured by Ohtsuka Electron Co., Ltd.), and the resolubility after lyophilization was visually evaluated after adding 10 mL of water to 50 mg of the lyophilized product. The results thereof are shown in Table 2 (the evaluation criteria are the same as in Table 1).

**Table 2 Average particle diameter change ratio before and after lyophilization in adding Macrogols in a void micelle and resolubility**

| Additives | Additive concentration | Particle diameter before lyophilization | Particle diameter after lyophilization | Average particle diameter change ratio before & after lyophilization | Resolubility |
|---|---|---|---|---|---|
| | (mg/mL) | (nm) | (nm) | | |
| Macrogol 400 | 20 | 77.7 | 145.1 | 1.87 | Average |
| Macrogol 1000 | 20 | 69.8 | 80.8 | 1.16 | Good |
| Macrogol 1540 | 20 | 79.2 | 83.4 | 1.05 | Good |
| Macrogol 4000 | 20 | 88.4 | 87.5 | 0.99 | Good |
| Macrogol 6000 | 20 | 94.0 | 79.8 | 0.85 | Good |

### Example 3 (investigation of effect exerted by adding Macrogols and maltose in void micelle)

PEG-PBLA12-50. PH. 50 % 500 mg was weighed in a screw tube bottle, and 50 mL of dichloromethane was added thereto and stirred to dissolve it. Next, the dichloromethane solution was concentrated up to 5 mL by blowing nitrogen gas, and 50 mL of water was added thereto and vigorously stirred for 30 minutes. Then, the stopper was opened, and the solution was vigorously stirred in a cold place for a whole day and night to prepare a polymer micelle. Thereafter, supersonic treatment was carried out, and maltose was added and dissolved in a concentration of 40 mg/mL. Further, various Macrogols shown in Table 3 were added and dissolved in a concentration of 20 mg/mL, and the solution was frozen in a dry ice-acetone freezing mixture to prepare a lyophilized preparation.

A micelle solution before lyophilization and a micelle solution obtained by lyophilizing the micelle solution and then redissolving it in water were measured for a particle size by means of the dynamic light scattering particle size meter (DLS-7000DH type, manufactured by Ohtsuka Electron Co., Ltd.), and the resolubility after lyophilization was visually evaluated after adding 10 mL of water to 50 mg of the lyophilized product. The results thereof are shown in Table 3.

**Table 3 Average particle diameter change ratio before and after lyophilization in adding Macrogols and maltose in void micelle and resolubility**

| Additives | Additive concentration | Particle diameter before lyophilization | Particle diameter after lyophilization | Average particle diameter change ratio before & after lyophilization | Resolubility |
|---|---|---|---|---|---|
| | (mg/mL) | (nm) | (nm) | | |
| Macrogol 400 | 20 | 101.6 | 196.2 | 1.93 | Average |
| Macrogol 1000 | 20 | 80.8 | 81.8 | 1.01 | Good |
| Macrogol 1540 | 20 | 99.5 | 109.4 | 1.10 | Good |
| Macrogol 4000 | 20 | 97.9 | 96.5 | 0.99 | Good |
| Macrogol 6000 | 20 | 105.7 | 98.5 | 0.93 | Good |

### Example 4 (investigation of effect exerted by adding saccharides and Macrogol 4000 in void micelle)

PEG-PBLA12-50. PH. 50 % 500 mg was weighed in a screw tube bottle, and 50 mL of dichloromethane was added thereto and stirred to dissolve it. Next, the dichloromethane solution was concentrated up to 5 mL by blowing nitrogen gas, and 50 mL of water was added thereto and vigorously stirred for 30 minutes. Then, the stopper was opened, and the solution was stirred in a cold place for a whole day and night to prepare a polymer micelle. Thereafter, supersonic treatment was carried out, and various saccharides shown in Table 4 and Macrogol 4000 were added and dissolved in a concentration of 20 to 40 mg/mL and a concentration of 0 to 40 mg/mL respectively. The solution was frozen in a dry ice-acetone freezing mixture to prepare a lyophilized preparation.

A micelle solution before lyophilization and a micelle solution obtained by lyophilizing the micelle solution and then redissolving it in water were measured for a particle size by means of the dynamic light scattering particle size meter (DLS-7000DH type, manufactured by Ohtsuka Electron Co., Ltd.), and the resolubility after lyophilization was visually evaluated after adding 10 mL of water to 50 mg of the lyophilized product. The results thereof are shown in Table 4.

**Table 4 Average particle diameter change ratio before and after lyophilization in adding saccharides and Macrogol 4000 in a void micelle and resolubility**

| Saccharides and concentration | Macrogol 4000 and concentration | Particle diameter before lyophilization | Particle diameter after lyophilization | Average particle diameter change ratio before and after lyophilization | Resolubility |
|---|---|---|---|---|---|
| (mg/mL) | (mg/mL) | (nm) | (nm) | | |
| Maltose (40mg/mL) | Not added | 94.3 | 118.5 | 1.26 | Average |
| Maltose (40mg/mL) | 10 | 96.9 | 110.2 | 1.14 | Good |
| Maltose (40mg/mL) | 20 | 102.3 | 103.5 | 1.01 | Good |
| Maltose (40mg/mL) | 40 | 93.9 | 103.3 | 1.10 | Good |
| Maltose (20mg/mL) | 20 | 90.6 | 101.7 | 1.12 | Good |
| Trehalose (40mg/mL) | Not added | 104.6 | 128.0 | 1.22 | Average |
| Trehalose (40mg/mL) | 10 | 101.3 | 118.3 | 1.17 | Good |
| Trehalose (40mg/mL) | 20 | 95.6 | 99.1 | 1.04 | Good |
| Trehalose (40mg/mL) | 40 | 90.9 | 109.4 | 1.20 | Good |
| Trehalose (20mg/mL) | 20 | 101.3 | 97.3 | 0.96 | Good |
| Fructose (40mg/mL) | 20 | 96.2 | 99.8 | 1.04 | Good |
| Lactose (40mg/mL) | 20 | 102.9 | 106.4 | 1.03 | Good |
| Xylitol (40mg/mL) | 20 | 89.7 | 126.0 | 1.40 | Good |

### Example 5 (investigation of effect exerted by adding saccharides and Macrogol 4000 in a paclitaxel micelle)

Paclitaxel 100 mg and PEG-PBLA12-50. PH. 50 % 500 mg were weighed in a screw tube bottle, and 50 mL of dichloromethane was added thereto and stirred to dissolve them. Next, the dichloromethane solution was concentrated up to 5 mL by blowing nitrogen gas, and 50 mL of a 5 % sodium chloride aqueous solution was added thereto and vigorously stirred for 30 minutes. Then, the stopper was opened, and the solution was vigorously stirred in a cold place for a whole day and night. After desalinating by means of ultrafiltration, supersonic treatment was carried out, and various saccharides shown in Table 5 and Macrogol 4000 were added and dissolved in a concentration of 40 mg/mL and a concentration of 10 to 30 mg/mL respectively. The solution was frozen in a dry ice-acetone freezing mixture to prepare a lyophilized preparation. Further, a preparation in which the saccharides and Macrogol 4000 were not added was prepared as a comparative lyophilized preparation.

A micelle solution before lyophilization and a micelle solution obtained by lyophilizing the micelle solution and then redissolving it in water were measured for a particle size by means of the dynamic light scattering particle size meter (DLS-7000DH type, manufactured by Ohtsuka Electron Co., Ltd.), and the resolubility after lyophilization was visually evaluated after adding 10 mL of water to 50 mg of the lyophilized product. The results thereof are shown in Table 5.

**Table 5 Average particle diameter change ratio before and after lyophilization in wadding saccharides and Macrogol 4000 in a paclitaxel micelle and resolubility**

| Saccharides and concentration | Macrogol 4000 and concentration | Particle diameter before lyophilization | Particle diameter after lyophilization | Average particle diameter change ratio before and after lyophilization | Resolubility |
|---|---|---|---|---|---|
| (mg/mL) | (mg/mL) | (nm) | (nm) | | |
| Maltose (40 mg/ml) | 20 | 159.6 | 209.6 | 1.32 | Good |
| Trehalose (40 mg/ml) | Not added | 160.1 | 408.5 | 2.55 | Average |
| Trehalose (40 mg/ml) | 10 | 161.5 | 261.7 | 1.62 | Good |
| Trehalose (40 mg/ml) | 20 | 171.3 | 202.4 | 1.18 | Good |
| Not added | 30 | 158.4 | 197.1 | 1.24 | Good |
| Not added | Not added | 164.9 | 445.3 | 2.70 | Bad |

### Example 6 (investigation of effect exerted by adding maltose and Macrogol 4000 in a paclitaxel micelle)

Paclitaxel 60 mg and PEG-PBLA12-50. PH. 50 % 300 mg were weighed in a screw tube bottle, and 30 mL of dichloromethane was added thereto and stirred to dissolve them. Next, the dichloromethane solution was concentrated up to 3 mL by blowing nitrogen gas, and 30 mL of a 40 mg/mL maltose aqueous solution was added thereto. The bottle was tightly stoppered and vigorously stirred in a refrigerator for 30 minutes. Then, the stopper was opened, and supersonic treatment was carried out while vigorously stirring in the refrigerator for a whole day and night. Further, Macrogol 4000 was added and dissolved in a concentration of 20 mg/mL, and the solution was sterilized, filtered and then frozen in a dry ice-acetone freezing mixture to prepare a lyophilized preparation.

A micelle solution before lyophilization and a micelle solution obtained by lyophilizing the micelle solution and then redissolving it in water were measured for a particle size by means of the dynamic light scattering particle size meter (DLS-7000DH type, manufactured by Ohtsuka Electron Co., Ltd.), and the resolubility after lyophilization was visually evaluated after adding 10 mL of water to 50 mg of the lyophilized product. The results thereof are shown in Table 6.

**Table 6 Average particle diameter change ratio before and after lyophilization in adding maltose and Macrogol 4000 in a paclitaxel micelle and resolubility**

| Particle diameter before lyophilization (nm) | Particle diameter after lyophilization (nm) | Average particle diameter change ratio before and after lyophilization | Resolubility |
|---|---|---|---|
| 119.0 | 139.5 | 1.17 | Good |

### Example 7 (cisplatin)

A polyethylene glycol-poly(α,β-aspartic acid) block polymer PEG-P(Asp)BP and a poly(α,β-aspartic acid) block homopolymer P(Asp)HP were dissolved in a cisplatin (hereinafter referred to as CDDP) aqueous solution of 15 mg/mL (5 mmmol/mL) so that a mole ratio (CDDP/Asp) of cisplatin to an Asp residue was 1.0, and the solution was shaken at 37°C for 72 hours to thereby prepare a micelle. The micelle solution thus obtained was refined by carrying out ultrafiltration through a membrane having a fractioned molecular weight of 100,000, and maltose and Macrogol 4000 were added to this refined micelle aqueous solution and dissolved in a concentration of 40 mg/mL and a concentration of 10 mg/mL respectively. The solution was frozen in a dry ice-acetone freezing mixture to prepare a lyophilized preparation.

A micelle solution before lyophilization and a micelle solution obtained by lyophilizing the micelle solution and then redissolving it in water were measured for a particle size by means of the dynamic light scattering particle size meter (DLS-7000DH type, manufactured by Ohtsuka Electron Co., Ltd.), and the resolubility after lyophilization was visually evaluated after adding 10 mL of water to 50 mg of the lyophilized product. The results thereof are shown in Table 7.

**Table 7 Average particle diameter change ratio before and after lyophilizing cisplatin and resolubility**

| Particle diameter before lyophilization (nm) | Particle diameter after lyophilization (nm) | Average particle diameter change ratio before and after lyophilization | Resolubility |
|---|---|---|---|
| 124.5 | 145.3 | 1.16 | Good |

### Example 8 (beraprost)

Beraprost 50 mg and PEG-PBLA12-50. PH. 50 % 300 mg were weighed in a screw tube bottle, and 30 mL of dichloromethane was added thereto and stirred to dissolve them. Next, the dichloromethane solution was concentrated up to 3 mL by blowing nitrogen gas, and 30 mL of a 40 mg/mL maltose aqueous solution was added thereto. The bottle was tightly stoppered and vigorously stirred in a refrigerator for 30 minutes. Then, the stopper was opened, and supersonic treatment was carried out while vigorously stirring in the refrigerator for a whole day and night. Further, Macrogol 4000 was added and dissolved in a concentration of 20 mg/mL, and the solution was sterilized, filtered and then frozen in a dry ice-acetone freezing mixture to prepare a lyophilized preparation.

A micelle solution before lyophilization and a micelle solution obtained by lyophilizing the micelle solution and then redissolving it in water were measured for a particle size by means of the dynamic light scattering particle size meter (DLS-7000DH type, manufactured by Ohtsuka Electron Co., Ltd.), and the resolubility after lyophilization was visually evaluated after adding 10 mL of water to 50 mg of the lyophilized product. The results thereof are shown in Table 8.

**Table 8 Average particle diameter change ratio before and after lyophilizing adreamycin and resolubility**

| Particle diameter before lyophilization (nm) | Particle diameter after lyophilization (nm) | Average particle diameter change ratio before and after lyophilization | Resolubility |
|---|---|---|---|
| 91.3 | 110.6 | 1.21 | Good |

Further, the present invention shall more specifically be explained below with reference to comparative production examples of drug-encapsulating polymer micelles and production examples thereof according to the present invention.

### Comparative Production Example 1 (process 1 for preparing a micelle of paclitaxel)

Paclitaxel 20 mg and polyethylene glycol (molecular weight: 12000)-co-50 % partially hydrolyzed polybenzyl aspartate (n = 50) (hereinafter referred to as PEG-PBLA12-50. PH. 50 %) 100 mg were weighed in a screw tube bottle, and 10 mL of dichloromethane was added thereto and stirred to dissolve them. Next, dichloromethane was volatilized by blowing nitrogen gas to dry up the solution. Further, 1 mL of dichloromethane was added thereto and slowly stirred so that the sample adhered on the tube wall was dissolved as well, whereby the residue was redissolved so that a homogeneous state was obtained. A 5 % sodium chloride aqueous solution 10 mL was added thereto, and the bottle was tightly stopper and vigorously stirred for 30 minutes. Then, the stopper was opened, and the solution was vigorously stirred in a cold place for a whole day and night. After desalinating by means of ultrafiltration, supersonic treatment (130 W, 1 sec Pulse, 10 minutes) was carried out, and a part of the sample was taken and measured for a particle size by means of the dynamic light scattering particle size meter (DLS-7000DH type, manufactured by Ohtsuka Electron Co., Ltd.). Further, maltose and Macrogol 4000 were added and dissolved in a concentration of 40 mg/mL and a concentration of 20 mg/mL respectively, and the solution was frozen in a dry ice-acetone freezing mixture to prepare a lyophilized preparation. The average particle diameter after the supersonic treatment was 97.5 nm.

Time passing up to the supersonic treating step was 32 hours.

### Comparative Production Example 2 (process 2 for preparing a micelle of paclitaxel)

Paclitaxel 60 mg and PEG-PBLA12-50. PH. 50 % 300 mg were weighed in a screw tube bottle, and 30 mL of dichloromethane was added thereto and stirred to dissolve them. Next, dichloromethane was volatilized by blowing nitrogen gas to dry up the solution. Further, 3 mL of dichloromethane was added thereto and slowly stirred so that the sample adhered on the tube wall was dissolved as well, whereby the residue was redissolved so that a homogeneous state was obtained. A 40 mg/mL maltose aqueous solution 30 mL was added thereto, and the bottle was tightly stoppered and vigorously stirred in a refrigerator for 30 minutes. Then, the stopper was opened, and the solution was vigorously stirred in the refrigerator for a whole day and night. Supersonic treatment (130 W, 1 sec Pulse, 10 minutes) was carried out, and a part of the sample was taken and measured for a particle size by means of the dynamic light scattering particle size meter (DLS-7000DH type, manufactured by Ohtsuka Electron Co., Ltd.). Further, Macrogol 4000 was added and dissolved in a concentration of 20 mg/mL, and the solution was sterilized, filtered and then frozen in a dry ice-acetone freezing mixture to prepare a lyophilized preparation.

The average particle diameter after the supersonic treatment was 111.4 nm.

Time passing up to the supersonic treating step was 25 hours.

### Comparative Production Example 3 (process 3 for preparing a micelle of beraprost)

Beraprost 30 mg and PEG-PBLA12-50. PH. 50 % 300 mg were weighed in a screw tube bottle, and 30 mL of dichloromethane was added thereto and stirred to dissolve them. Next, dichloromethane was volatilized by blowing nitrogen gas to dry up the solution. Further, 3 mL of dichloromethane was added thereto and slowly stirred so that the sample adhered on the tube wall was dissolved as well, whereby the residue was redissolved so that a homogeneous state was obtained. A 5 % sodium chloride aqueous solution 30 mL was added thereto, and the bottle was tightly stoppered and vigorously stirred at a room temperature for 60 minutes. Then, the stopper was opened, and the solution was vigorously stirred at a room temperature for a whole day and night. Supersonic treatment (130 W, 1 sec Pulse, 10 minutes) was carried out, and a part of the sample was taken and measured for a particle size by means of the dynamic light scattering particle size meter (DLS-7000DH type, manufactured by Ohtsuka Electron Co., Ltd.). Further, the solution was desalinated by means of ultrafiltration, sterilized and then filtered to obtain a preparation.

The average particle diameter after the supersonic treatment was 72.2 nm.

Time passing up to the supersonic treating step was 32 hours.

### Comparative Production Example 4 (dialysis)

Paclitaxel 10 mg and PEG-PBLA12-50. PH. 50 % were dissolved in 5 mL of DMSO (dimethylsulfoxide), and the solution was dialyzed to 100 mL of a physiological salt solution through a dialysis membrane (fractioned molecular weight: 12-14000) for 16 hours.

As a result thereof, the dialyzed sample was precipitated and did not have a micelle form.

### Comparative Production Example 5 (dialysis)

Paclitaxel 10 mg and PEG-PBLA12-50. PH. 50 % were dissolved in 5 mL of DMF (dimethylformamide), and the solution was dialyzed to 100 mL of a physiological salt solution through a dialysis membrane (fractioned molecular weight: 12-14000) for 16 hours.

As a result thereof, the dialyzed sample was precipitated and did not have a micelle form.

### Production Example 1 (process 1 for preparing a micelle of paclitaxel according to the present invention)

PEG-PBLA12-50. PH. 50 % 300 mg was weighed in a screw tube bottle, and a 40 mg/mL maltose aqueous solution 30 mL was added thereto and stirred to prepare a dispersion. The dispersion was cooled down to 4°C while further stirring. Further, a 20 mg/mL paclitaxel dichloromethane solution 3 mL was added thereto, and the mixture was stirred in a refrigerator for 16 hours without tightly stoppering. Then, supersonic treatment (130 W, 1 sec Pulse, 10 minutes) was carried out, and a part of the sample was taken and measured for a particle size by means of the dynamic light scattering particle size meter (DLS-7000DH type, manufactured by Ohtsuka Electron Co., Ltd.). Further, the solution was sterilized, filtered and then frozen in a dry ice-acetone freezing mixture to prepare a lyophilized preparation.

The average particle diameter after the supersonic treatment was 107.3 nm.

Time passing up to the supersonic treating step was 19 hours.

### Production Example 2 (process 2 for preparing a micelle of paclitaxel according to the present invention)

PEG-PBLA12-50. PH. 50 % 300 mg was weighed in a screw tube bottle, and a 40 mg/mL maltose aqueous solution 30 mL was added thereto and stirred to prepare a dispersion. The dispersion was cooled down to 4°C while further stirring. Further, a 20 mg/mL paclitaxel dichloromethane solution 3 mL was added thereto, and the mixture was stirred in a refrigerator for 16 hours without tightly stoppering. Then, supersonic treatment (130 W, 1 sec Pulse, 10 minutes) was carried out, and a part of the sample was taken and measured for a particle size by means of the dynamic light scattering particle size meter (DLS-7000DH type, manufactured by Ohtsuka Electron Co., Ltd.). Further, Macrogol 4000 was added and dissolved in a concentration of 20 mg/mL, and the solution was sterilized, filtered and then frozen in a dry ice-acetone freezing mixture to prepare a lyophilized preparation.

The average particle diameter after the supersonic treatment was 107 nm.

Time passing up to a supersonic treating step was 19 hours.

### Production Example 3 (process 3 for preparing a micelle of beraprost according to the present invention)

PEG-PBLA12-50. PH. 50 % 300 mg was weighed in a screw tube bottle, and a 5 % sodium chloride aqueous solution 30 mL was added thereto and stirred to prepare a dispersion. Further, a 10 mg/mL beraprost dichloromethane solution 3 mL was added thereto, and the mixture was then vigorously stirred at a room temperature for a whole day and night. Supersonic treatment (130 W, 1 sec Pulse, 10 minutes) was carried out, and a part of the sample was taken and measured for a particle size by means of the dynamic light scattering particle size meter (DLS-7000DH type, manufactured by Ohtsuka Electron Co., Ltd.). Then, the solution was desalinated by means of ultrafiltration, sterilized and filtered to obtain a preparation.

The average particle diameter after the supersonic treatment was 72.1 nm.

Time passing up to a supersonic treating step was 25 hours.

### Industrial Applicability

According to the present invention, provided are a composition capable of providing a stable aqueous medical preparation which does not substantially cause coagulation between micelle particles when a drug-encapsulating polymer micelle staying in a lyophilized state is redissolved in water, and a process in which the composition can conveniently be produced.

Accordingly, the present invention can be applied to the medical field, particularly the medicinal production industry.

## Claims

1. An aqueous composition comprising a drug-encapsulating polymer micelle for preparing a lyophilized preparation of the drug-encapsulating polymer micelle, wherein:
(A) the composition further comprises at least one stabilizing agent selected from the group consisting of polyethylene glycol and a combination of saccharides and polyethylene glycol and
(B) the above drug-encapsulating polymer micelle is formed from a block copolymer having in the molecule, a hydrophilic polymer segment and a polymer segment which is hydrophobic or chargeable or which comprises the repetitive units of both of them, and it is a substantially spherical core-shell type micelle in which the drug is carried principally in a core part and in which a shell part is constituted by the above hydrophilic polymer segment.

2. The aqueous composition according to Claim 1, wherein the stabilizing agent is selected from the group consisting of saccharides which are maltose, trehalose, xylitol, glucose, sucrose, fructose, lactose, mannitol and dextrin and polyethylene glycol having a molecular weight of about 1000 to about 35000.

3. The aqueous composition according to Claim 1, wherein the hydrophilic polymer segment is a polyethylene glycol segment.

4. The aqueous composition according to Claim 3, wherein the polyethylene glycol segment has 10 to 2500 oxyethylene repetitive units.

5. The aqueous composition according to Claim 1, wherein the block copolymer is represented by Formula (I) or (II): 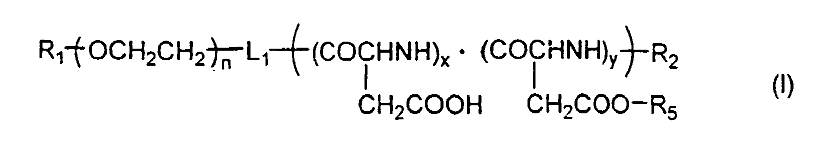 or 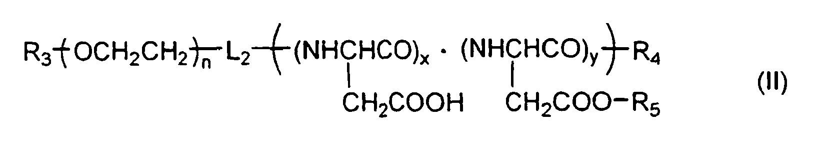 wherein
R₁ and R₃ each represent independently a hydrogen atom or a lower alkyl group substituted or not substituted with a functional group which may be protected;
R₂ represents a hydrogen atom, a saturated or unsaturated C₁ to C₂₉ aliphatic carbonyl group or an arylcarbonyl group;
R₄ represents a hydroxyl group, a saturated or unsaturated C₁ to C₃₀ aliphatic oxy group or an aryl-lower alkyloxy group;
R₅ represents a phenyl group, a C₁ to C₄ alkyl group or a benzyl group;
L₁ and L₂ each represent independently a linkage group;
n is an integer of 10 to 2500;
x and y are different or the same and are an integer in which the total of them is 10 to 300; either one of x and y is 0 or x to y falls in a range of 7 : 3 to 1 : 3; and when both are present, x and y each are present at random.

6. The aqueous composition according to Claim 1, wherein the drug is selected from the group consisting of anticancer drugs including paclitaxel, topotecan, camptothecine, adriamycin, daunomycin, methotrexate, mitomycin C, docetaxel and binclestin; polyene base antibiotics including anphoterisis B and nystatin; prostaglandins and derivatives thereof.

7. A drug-encapsulating polymer micelle preparation staying in a lyophilized form, wherein:
(a) the preparation comprises at least one stabilizing agent selected from the group consisting of polyethylene glycol and a combination of saccharides and polyethylene glycol as an additional component,
(b) the above drug-encapsulating polymer micelle is formed from a block copolymer having in the molecule, a hydrophilic polymer segment and a polymer segment which is hydrophobic or chargeable or which comprises the repetitive units of both of them, and it is a core-shell type micelle in which the drug is carried principally in a core part and in which a shell part is constituted by the above hydrophilic polymer segment and
(c) a drug-encapsulating polymer micelle solution which is homogeneously dispersed or solubilized is formed when the preparation is mixed with an aqueous medium.

8. The preparation according to Claim 7, wherein the stabilizing agent is selected from the group consisting of saccharides which are maltose, trehalose, xylitol, glucose, sucrose, fructose, lactose, mannitol and dextrin and polyethylene glycol having a molecular weight of about 1000 to about 35000.

9. The preparation according to Claim 7, wherein the hydrophilic polymer segment is a polyethylene glycol segment.

10. The preparation according to Claim 9, wherein the polyethylene glycol segment has 10 to 2500 oxyethylene repetitive units.

11. A process for producing a drug-encapsulating polymer micelle, comprising the steps of:
(A) preparing an aqueous dispersion comprising a block copolymer having a hydrophilic segment and a polymer segment which is hydrophobic or chargeable or which comprises the repetitive units of both of them and at least one additive selected from the group consisting of saccharides, inorganic salts and polyethylene glycol,
(B) preparing an organic solution of a fat-soluble drug using a water-immiscible organic solvent,
(C) mixing the aqueous dispersion and the organic solution each obtained in the step (A) and the step (B) and volatilizing the organic solvent while stirring the mixed solution thus obtained to prepare an aqueous dispersion or an aqueous composition of a drug-encapsulating polymer micelle and
(D) adding at least one additive selected from the group consisting of saccharides and polyethylene glycol to the dispersion of the drug-encapsulating polymer micelle described above and adding polyethylene glycol without fail when polyethylene glycol is not added in the step (A).

12. The process according to Claim 11, wherein the hydrophilic polymer segment is a polyethylene glycol segment.

13. The process according to Claim 11, wherein the block copolymer is represented by Formula (I) or (II): 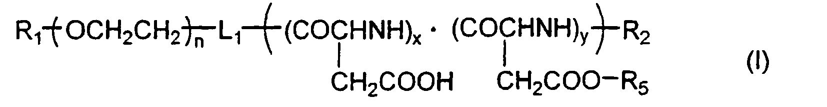 or 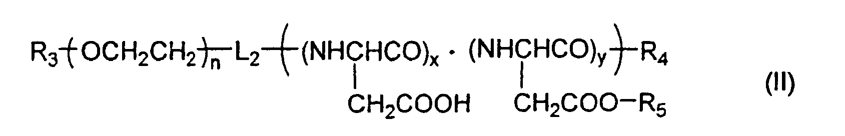 wherein
R₁ and R₃ each represent independently a hydrogen atom or a lower alkyl group substituted or not substituted with a functional group which may be protected;
R₂ represents a hydrogen atom, a saturated or unsaturated C₁ to C₂₉ aliphatic carbonyl group or an arylcarbonyl group;
R₄ represents a hydroxyl group, a saturated or unsaturated C₁ to C₃₀ aliphatic oxy group or an aryl-lower alkyloxy group;
R₅ represents a phenyl group, a C₁ to C₄ alkyl group or a benzyl group;
L₁ and L₂ each represent independently a linkage group;
n is an integer of 10 to 2500;
x and y are different or the same and are an integer in which the total of them is 10 to 300; either one of x and y is 0 or x to y falls in a range of 7 : 3 to 1 : 3; and when both are present, x and y each are present at random.

14. The process according to Claim 11, wherein the saccharides are selected from the group consisting of maltose, trehalose, xylitol, glucose, sucrose, fructose, lactose, mannitol and dextrin; or the inorganic salts are selected from the group consisting of sodium chloride, potassium chloride, magnesium chloride and calcium chloride; or polyethylene glycol is selected from the group consisting of polyethylene glycols having a molecular weight of about 1000 to about 35000.

15. The process according to Claim 11, wherein the fat-soluble drug is selected from the group consisting of anticancer drugs including paclitaxel, topotecan, camptothecine, cisplatin, adriamycin, daunomycin, methotrexate, mitomycin C, docetaxel and, binclestin; polyene base antibiotics including anphoterisis B and nystatin; prostaglandins and derivatives thereof.

16. A process for producing a drug-encapsulating polymer micelle preparation staying in a lyophilized form comprising the steps of:
(A) preparing an aqueous dispersion comprising a block copolymer having a hydrophilic segment and a hydrophobic segment and at least one additive selected from the group consisting of polyethylene glycol and a combination of saccharides, inorganic salts and polyethylene glycol,
(B) preparing an organic solution of a fat-soluble drug using a water-immiscible organic solvent,
(C) mixing the aqueous dispersion and the organic solution each obtained in the step (A) and the step (B) and volatilizing the organic solvent while stirring the mixed solution thus obtained to prepare an aqueous dispersion or an aqueous composition of a drug-encapsulating polymer micelle and
(E) lyophilizing the aqueous dispersion or the aqueous composition of the drug-encapsulating polymer micelle obtained in the step (C).

## Patentansprüche

1. Wässrige Zusammensetzung, die eine Wirkstoff-verkapselnde Polymermizelle umfasst, zur Herstellung einer lyophilisierten Präparation der Wirkstoff-verkapselnden Polymermizelle, wobei
(A) die Zusammensetzung außerdem wenigstens ein Stabilisierungsmittel, ausgewählt aus der Gruppe, bestehend aus Polyethylenglykol und einer Kombination aus Sacchariden und Polyethylenglykol, umfasst und
(B) die obige Wirkstoff-verkapselnde Polymermizelle aus einem Blockcopolymer, das im Molekül ein hydrophiles Polymersegment und ein Polymersegment, das hydrophob oder aufladbar ist, oder das die Repetiereinheiten von beiden von diesen umfasst, hat, besteht, die eine im Wesentlichen sphärische Mizelle vom Kern-Mantel-Typ ist, in welcher der Wirkstoff prinzipiell in einem Kernteil getragen wird und in welcher ein Mantelteil durch das obige hydrophile Polymersegment gebildet wird.

2. Wässrige Zusammensetzung nach Anspruch 1, wobei das Stabilisierungsmittel aus der Gruppe, bestehend aus Sacchariden, die Maltose, Trehalose, Xylit, Glucose, Saccharose, Fruktose, Laktose, Mannit und Dextrin sind, und Polyethylenglykol, das ein Molekulargewicht von etwa 1000 bis etwa 35000 hat, ausgewählt ist.

3. Wässrige Zusammensetzung nach Anspruch 1, wobei das hydrophile Polymersegment ein Polyethylenglykolsegment ist.

4. Wässrige Zusammensetzung nach Anspruch 3, wobei das Polyethylenglykolsegment 10 bis 2500 Oxyethylen-Repetiereinheiten hat.

5. Wässrige Zusammensetzung nach Anspruch 1, wobei das Blockcopolymer durch Formel (I) oder (II) dargestellt wird: 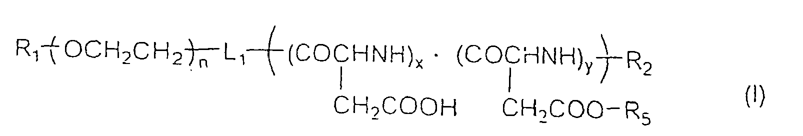 oder 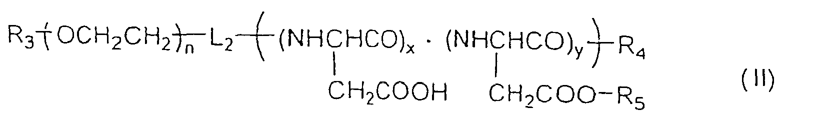 wobei
R₁ und R₃ jeweils unabhängig ein Wasserstoffatom oder eine Niederalkylgruppe darstellen, die mit einer funktionellen Gruppe, welche geschützt sein kann, substituiert ist oder nicht substituiert ist;
R₂ ein Wasserstoffatom, eine gesättigte oder ungesättigte aliphatische C₁-C₂₉-Carbonylgruppe oder eine Arylcarbonylgruppe darstellt;
R₄ eine Hydroxylgruppe, eine gesättigte oder ungesättigte aliphatische C₁-C₃₀-Oxygruppe oder eine Aryl-Niederalkyloxygruppe darstellt;
R₅ eine Phenylgruppe, eine C₁-C₄-Alkylgruppe oder eine Benzylgruppe darstellt;
L₁ und L₂ jeweils unabhängig eine Verknüpfungsgruppe darstellen;
n eine ganze Zahl von 10 bis 2500 ist;
x und y unterschiedlich oder gleich sind und eine ganze Zahl sind, wobei die Summe von ihnen 10 bis 300 ist; wobei entweder eines von x und y 0 ist oder x zu y in einen Bereich von 7:3 bis 1:3 fällt; und wenn beide vorliegen, x und y jeweils statistisch vorliegen.

6. Wässrige Zusammensetzung nach Anspruch 1, wobei der Wirkstoff aus der Gruppe bestehend aus Antikrebsmitteln, einschließlich Paclitaxel, Topotecan, Camptothecin, Adriamycin, Daunomycin, Methotrexat, Mitomycin C, Docetaxel und Binclestin; Antibiotika auf Polyenbasis, einschließlich Anphoterisis B und Nystatin; Prostaglandinen und Derivaten davon, ausgewählt ist.

7. Eine Wirkstoff-verkapselnde Polymermizellen-Präparation, die in lyophilisierter Form bleibt, wobei
(a) die Präparation wenigstens ein Stabilisierungsmittel, ausgewählt aus der Gruppe, bestehend aus Polyethylenglykol und einer Kombination aus Sacchariden und Polyethylenglykol ausgewählt ist, als zusätzliche Komponente umfasst;
(b) die obige Wirkstoff-verkapselnde Polymermizelle aus einem Blockcopolymer gebildet ist, das im Molekül ein hydrophiles Polymersegment und ein Polymersegment, das hydrophob oder aufladbar ist, oder das die Repetiereinheiten von beiden von diesen umfasst, hat, und die eine Mizelle vom Kern-Mantel-Typ ist, in welcher der Wirkstoff prinzipiell in einem Kernteil getragen wird und in welcher ein Mantelteil durch das obige hydrophile Polymersegment gebildet wird, und
(c) eine Wirkstoff-verkapselnde Polymermizellen-Lösung, die homogen dispergiert oder solubilisiert ist, gebildet wird, wenn die Präparation mit einem wässrigen Medium vermischt wird.

8. Präparation nach Anspruch 7, wobei das Stabilisierungsmittel aus der Gruppe, bestehend aus Sacchariden, die Maltose, Trehalose, Xylit, Glukose, Saccharose, Fruktose, Laktose, Mannit und Dextrin sind, und Polyethylenglykol, das ein Molekulargewicht von etwa 1000 bis etwa 35000 hat, ausgewählt ist.

9. Präparation nach Anspruch 7, wobei das hydrophile Polymersegment ein Polyethylenglykolsegment ist.

10. Präparation nach Anspruch 9, wobei das Polyethylenglykolsegment 10 bis 2500 Oxyethylen-Repetiereinheiten hat.

11. Verfahren zur Herstellung einer Wirkstoff-verkapselnden Polymermizelle, umfassend die Schritte:
(A) Herstellen einer wässrigen Dispersion, die ein Blockcopolymer, das ein hydrophiles Segment und ein Polymersegment, welches hydrophob oder aufladbar ist oder welches die Repetiereinheiten von beiden umfasst, hat, und wenigstens ein Additiv, ausgewählt aus der Gruppe, bestehend aus Sacchariden, anorganischen Salzen und Polyethylenglykol, umfasst;
(B) Herstellen einer organischen Lösung eines fettlöslichen Wirkstoffs unter Verwendung eines mit Wasser nicht mischbaren organischen Lösungsmittels;
(C) Vermischen der wässrigen Dispersion und der organischen Lösung, die in Schritt (A) bzw. Schritt (B) erhalten wurden, und Verflüchtigen des organischen Lösungsmittels unter Rühren der gemischten Lösung, die so erhalten wurde, unter Herstellung einer wässrigen Dispersion oder einer wässrigen Zusammensetzung einer Wirkstoff-verkapselnden Polymermizelle und
(D) Zusetzen wenigstens eines Additivs, ausgewählt aus der Gruppe, bestehend aus Sacchariden und Polyethylenglykol, zu der Dispersion der Wirkstoff-verkapselnden Polymermizelle, die oben beschrieben wurde, und Zugeben von Polyethylenglykol ohne Fehlschlag, wenn Polyethylenglykol im Schritt (A) nicht zugegeben wird.

12. Verfahren nach Anspruch 11, wobei das hydrophile Polymersegment ein Polyethylenglykolsegment ist.

13. Verfahren nach Anspruch 11, wobei das Blockcopolymer durch Formel (I) oder (II) dargestellt wird: 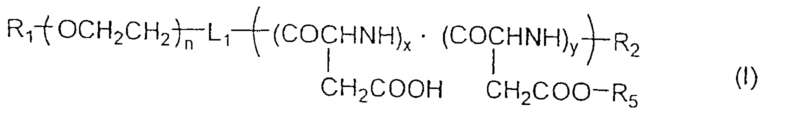 oder 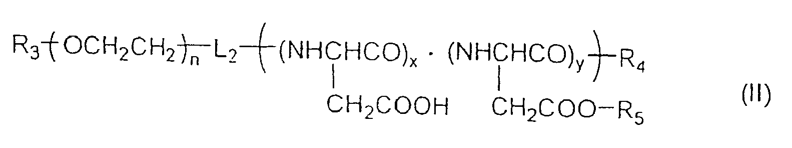 wobei
R₁ und R₃ jeweils unabhängig voneinander ein Wasserstoffatom oder eine Niederalkylgruppe, die mit einer funktionellen Gruppe, die geschützt sein kann, substituiert ist oder nicht substituiert ist, darstellen;
R₂ ein Wasserstoffatom, eine gesättigte oder ungesättigte aliphatische C₁-C₂₉-Carbonylgruppe oder eine Arylcarbonylgruppe darstellt;
R₄ eine Hydroxylgruppe, eine gesättigte oder ungesättigte aliphatische C₁-C₃₀-Oxygruppe oder eine Aryl-Niederalkyloxygruppe darstellt;
R₅ eine Phenylgruppe, eine C₁-C₄-Alkylgruppe oder eine Benzylgruppe darstellt;
L₁ und L₂ jeweils unabhängig eine Verknüpfungsgruppe darstellen;
n eine ganze Zahl von 10 bis 2500 ist;
x und y voneinander verschieden sind oder gleich sind und eine ganze Zahl sind, wobei die Summe von ihnen 10 bis 300 ist; entweder eines von x und y 0 ist oder x zu y in den Bereich von 7:3 bis 1:3 fällt; und wenn beide vorliegen, x und y jeweils statistisch vorliegen.

14. Verfahren nach Anspruch 11, wobei die Saccharide aus der Gruppe, bestehend aus Maltose, Trehalose, Xylit, Glukose, Saccharose, Fruktose, Laktose, Mannit und Dextrin ausgewählt werden oder die anorganischen Salze aus der Gruppe bestehend aus Natriumchlorid, Kaliumchlorid, Magnesiumchlorid und Calciumchlorid ausgewählt werden oder Polyethylenglykol aus der Gruppe bestehend aus Polyethylenglykolen, die ein Molekulargewicht von etwa 1000 bis etwa 35000 haben, ausgewählt wird.

15. Verfahren nach Anspruch 11, wobei der fettlösliche Wirkstoff aus der Gruppe bestehend aus Antikrebswerkstoffen, einschließlich Paclitaxel, Topotecan, Camptothecin, Cisplatin, Adriamycin, Daunomycin, Methotrexat, Mitomycin C, Docetaxel und Binclestin; Antibiotika auf Polyenbasis, einschließlich Anphoterisis B und Nystatin; Prostaglandinen und Derivaten davon, ausgewählt wird.

16. Verfahren zur Herstellung einer Wirkstoff-verkapselnden Polymermizellen-Präparation, die in lyophilisierter Form bleibt, umfassend die Schritte:
(A) Herstellen einer wässrigen Dispersion, die ein Blockcopolymer, das ein hydrophiles Segment und ein hydrophobes Segment hat, und wenigstens ein Additiv, ausgewählt aus der Gruppe, bestehend aus Polyethylenglykol und einer Kombination von Sacchariden, anorganischen Salzen und Polyethylenglykol, umfasst;
(B) Herstellen einer organischen Lösung eines fettlöslichen Wirkstoffs unter Verwendung eines mit Wasser nicht mischbaren organischen Lösungsmittels;
(C) Vermischen der wässrigen Dispersion und der organischen Lösung, die in Schritt (A) bzw. in Schritt (B) erhalten wurden, und Verflüchtigen des organischen Lösungsmittels unter Rühren der gemischten Lösung, die so erhalten wurde, unter Herstellung einer wässrigen Dispersion oder einer wässrigen Zusammensetzung einer Wirkstoff-verkapselnden Polymermizelle und
(E) Lyophilisieren der wässrigen Dispersion oder der wässrigen Zusammensetzung der Wirkstoff-verkapselnden Polymermizelle, die in Schritt (C) erhalten wurde.

## Revendications

1. Composition aqueuse comprenant une micelle de polymère pour l'encapsulation de médicaments pour la préparation d'une préparation lyophilisée de la micelle de polymère pour l'encapsulation de médicaments, où
(A) la composition comprend de plus au moins un agent stabilisant pris dans le groupe constitué par le polyéthylène glycol et une combinaison de saccharides et de polyéthylène glycol et
(B) la micelle de polymère pour l'encapsulation de médicaments ci-dessus est formée à partir d'un copolymère à blocs renfermant dans la molécule un segment polymère hydrophile et un segment polymère qui est hydrophobe ou susceptible à être chargée, ou qui comprend les motifs répétitifs des deux, et la micelle est essentiellement sphérique de type coeur-enveloppe dans laquelle le médicament est porté principalement par une partie coeur et dans laquelle une partie enveloppe est constituée par le segment polymère hydrophile ci-dessus.

2. Composition aqueuse selon la revendication 1, dans laquelle l'agent stabilisant est pris dans le groupe constitué par des saccharides qui sont le maltose, le tréhalose, le xylitol, le glucose, le sucrose, le fructose, le lactose, le mannitol et la dextrine et le polyéthylène glycol ayant une masse moléculaire d'environ 1000 à environ 35000.

3. Composition aqueuse selon la revendication 1, dans laquelle le segment polymère hydrophile est un segment polyéthylène glycol.

4. Composition aqueuse selon la revendication 3, dans laquelle le segment polyéthylène glycol présente de 10 à 2500 motifs répétitifs oxyéthylène.

5. Composition aqueuse conformément à la revendication 1, dans laquelle le copolymère à blocs est représenté par la formule (I) ou (II) : 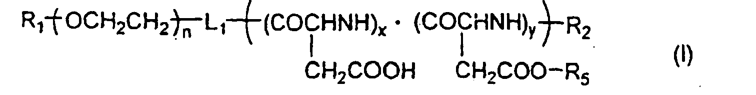 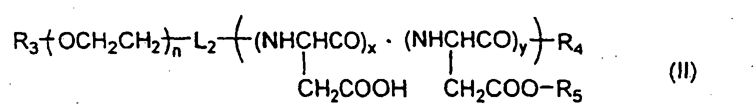 dans lesquelles
R₁ et R₃, indépendamment, chacun représente un atome d'hydrogène ou un groupe alkyle inférieur substitué ou non substitué par un groupe fonctionnel qui peut être protégé ;
R₂ représente un atome d'hydrogène, un groupe arylcarbonyle ou groupe carbonyle en C₁-C₂₉ aliphatique saturé ou insaturé ;
R₄ représente un groupe hydroxyle, un groupe arylalkyloxy inférieur ou un groupe oxy en C₁-C₃₀ aliphatique saturé ou insaturé ;
R₅ représente un groupe phényle, un groupe alkyle en C₁-C₄ ou un groupe benzyle ;
L₁ et L₂ indépendamment chacun représente un groupe de liaison ;
n est un entier de 10 à 2500 ;
x et y sont différents ou identiques et sont un entier dans lequel leur total est de 10 à 300 ; soit un des x et y vaut 0, soit x à y se situe dans le domaine de 7:3 à 1:3 ; et lorsque les deux sont présents, x et y chacun est présent au hasard.

6. Composition aqueuse selon la revendication 1, dans laquelle le médicament est pris dans le groupe constitué par des médicaments anticancéreux comprenant le paclitaxel, le topotécane, la camptothécine, l'adriamycine, la daunomycine, le méthotrexate, la mitomycine C, le docétaxel et la binclestine ; des antibiotiques à base de polyène comprenant l'anphoterisis B et la nystatine ; des prostaglandines et leurs dérivés.

7. Préparation d'une micelle de polymère pour l'encapsulation de médicaments demeurant sous forme lyophilisée, dans laquelle :
(a) la préparation comprend au moins un agent stabilisant pris dans le groupe constitué par le polyéthylène glycol et une combinaison de saccharides et de polyéthylène glycol en tant que composant supplémentaire,
(b) la micelle de polymère pour l'encapsulation de médicaments ci-dessus est formée par un copolymère à blocs ayant dans la molécule un segment polymère hydrophile et un segment polymère qui est hydrophobe ou susceptible à être chargé ou qui comprend les motifs répétitifs des deux, et c'est une micelle de type coeur-enveloppe dans laquelle le médicament est porté principalement par une partie coeur et dans laquelle une partie enveloppe est constituée par le segment polymère hydrophile ci-dessus, et
(c) une solution de micelle de polymère pour l'encapsulation de médicaments ci-dessus, qui est dispersée de façon homogène ou solubilisée est formée lorsque la préparation est mélangée avec un milieu aqueux.

8. Préparation selon la revendication 7, dans laquelle l'agent stabilisant est pris dans le groupe constitué par des saccharides qui sont le maltose, le tréhalose, le xylitol, le glucose, le sucrose, le fructose, le lactose, le mannitol et la dextrine et le polyéthylène glycol d'une masse moléculaire d'environ 1000 à environ 35000.

9. Préparation selon la revendication 7, dans laquelle le segment polymère hydrophile est un segment polyéthylène glycol.

10. Préparation selon la revendication 9, dans laquelle le segment polyéthylène glycol présente de 10 à 2500 motifs répétitifs oxyéthylène.

11. Procédé de préparation d'une micelle de polymère pour l'encapsulation de médicaments, comprenant les étapes de
(A) préparation d'une dispersion aqueuse comprenant un copolymère à blocs ayant un segment hydrophile et un segment polymère qui est hydrophobe ou susceptible à être chargé ou qui comprend les motifs répétitifs des deux et au moins un additif pris dans le groupe constitué par des saccharides, des sels inorganiques et le polyéthylène glycol,
(B) préparation d'une solution organique d'un médicament liposoluble utilisant un solvant organique non-miscible à l'eau,
(C) mélange de la dispersion aqueuse et de la solution organique, chacune étant obtenue à l'étape (A) et à l'étape (B), et volatilisation du solvant organique tout en agitant la solution mélangée ainsi obtenue afin de préparer une dispersion aqueuse ou une composition aqueuse d'une micelle de polymère pour l'encapsulation de médicaments et
(D) ajout d'au moins un additif pris dans le groupe constitué par des saccharides et le polyéthylène glycol à la dispersion de la micelle de polymère pour l'encapsulation de médicaments décrite ci-dessus et sans omettre d'ajouter le polyéthylène glycol lorsque le polyéthylène glycol n'est pas ajouté à l'étape (A).

12. Procédé selon la revendication 11, dans lequel le segment polymère hydrophile est un segment polyéthylène glycol.

13. Procédé selon la revendication 11, dans lequel le copolymère à blocs est représenté par la formule (I) ou (II) : 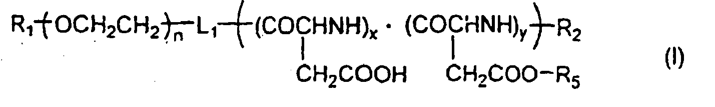 ou 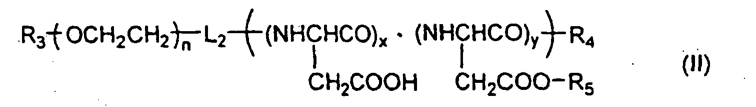 dans laquelle
R₁ et R₃, indépendamment, chacun représente un atome d'hydrogène ou un groupe alkyle inférieur substitué ou non substitué par un groupe fonctionnel qui peut être protégé ;
R₂ représente un atome d'hydrogène, un groupe arylcarbonyle ou un groupe carbonyle en C₁-C₂₉ aliphatique saturé ou insaturé ;
R₄ représente un groupe hydroxyle, un groupe aryl-alkyloxy inférieur ou un groupe oxy en C₁-C₃₀ aliphatique saturé ou insaturé ;
R₅ représente un groupe phényle, un groupe alkyle en C₁-C₄ ou un groupe benzyle ;
L₁ et L₂ indépendamment chacun représente un groupe de liaison ;
n est un entier de 10 à 2500 ;
x et y sont différents ou identiques et sont un entier
où leur total est de 10 à 300 ; soit un des x et y vaut 0, soit x à y se situe dans le domaine de 7 : 3 à 1 : 3 ; et lorsque les deux sont présents, x et y chacun est présent au hasard.

14. Procédé selon la revendication 11, dans lequel les saccharides sont pris dans le groupe constitué par le maltose, le tréhalose, le xylitol, le glucose, le sucrose, le fructose, le lactose, le mannitol et la dextrine ; ou les sels inorganiques sont pris dans le groupe constitué par le chlorure de sodium, le chlorure de potassium, le chlorure de magnésium et le chlorure de calcium ; ou le polyéthylène glycol est pris dans le groupe constitué par les polyéthylène glycols d'une masse moléculaire d'environ 1000 à environ 35000.

15. Procédé selon la revendication 11, dans lequel le médicament liposoluble est pris dans le groupe constitué par des médicaments anticancéreux comprenant le paclitaxel, le topotécane, la camptothécine, la cispatine, l'adriamycine, la daunomycine, le méthotrexate, la mitomycine C, le docétaxel et la binclestine ; des antibiotiques à base de polyène comprenant l'anphoterisis B et la nystatine ; des prostaglandines et leurs dérivés.

16. Procédé pour la préparation d'une micelle de polymère pour l'encapsulation de médicaments demeurant sous forme lyophilisée, comprenant les étapes de :
(A) préparation d'une dispersion aqueuse comprenant un copolymère à blocs ayant un segment hydrophile et un segment hydrophobe et au moins un additif pris dans le groupe constitué par le polyéthylène glycol et une combinaison de saccharides, de sels inorganiques et de polyéthylène glycol,
(B) préparation d'une solution organique d'un médicament liposoluble en utilisant un solvant organique non-miscible à l'eau,
(C) mélange de la dispersion aqueuse et de la solution organique, chacune étant obtenue à l'étape (A) et à l'étape (B), et volatilisation du solvant organique tout en agitant la solution mélangée ainsi obtenue afin de préparer une dispersion aqueuse ou une composition aqueuse d'une micelle de polymère pour l'encapsulation de médicaments et
(E) lyophilisation de la dispersion aqueuse ou de la composition aqueuse de la micelle de polymère pour l'encapsulation de médicaments obtenue à l'étape (C).
